# EUROPEAN PATENT APPLICATION

(11) **EP 4 667 018 A2**
(43) Date of publication of application: **24.12.2025**
(21) Application number: 25214725.1
(22) Date of filing: 06.11.2024
(51) Int. Cl.: A61K 49/22

(54) **ACOUSTIC MATCHING MEMBER AND METHOD FOR CAPTURING MEDICAL IMAGE**

(30) Priority: 21.11.2023 JP 2023197654
(62) Divisional of application: 24211269.6
(71) Applicant: FUJIFILM Corporation, Tokyo Tokyo 106-8620 (JP)
(72) Inventor: HORIUCHI, Hisatsugu, Kanagawa (JP); NAKAYAMA, Hiroki, Kanagawa (JP); SAITO, Sayaka, Kanagawa (JP); NOBUYAMA, Lisako, Kanagawa (JP); KOBAYASHI, Takeyasu, Kanagawa (JP)
(74) Representative: Dehns Germany Partnerschaft mbB

(57) **Abstract**

An acoustic matching member (80, 82) that is applied to a body surface in a case where an ultrasound image is captured, wherein the acoustic matching member (80, 82) is a mixed solution containing: water; and a hydrophilic polymer having a hydrophilic functional group and having 8 or more carbon atoms, and wherein a viscosity of the acoustic matching member (80, 82) is 5 mPa·s or more and 20000 mPa·s or less.

## Description

### BACKGROUND

### Technical Field

The present disclosure relates to an acoustic matching member and a method for capturing a medical image.

### Related Art

An ultrasound image capturing apparatus that captures an ultrasound image of a subject is known. As a method of capturing an ultrasound image using an ultrasound image capturing apparatus, for example, there is known an image capturing method of capturing an ultrasound image in a state where a subject is compressed by a compression member. As such an imaging method, JP2009-82399A discloses an image capturing method of capturing an ultrasound image of a breast in a compressed state using a mammography apparatus that captures a radiographic image with the breast in a compressed state by a compression member as a subject.

In a case where the ultrasound image is captured, the body surface in a state where the acoustic matching member is applied is scanned with an ultrasound probe. It is desirable that the acoustic matching member spreads on the body surface and remains on the body surface. In addition, a gel-like acoustic matching member such as echo jelly used in the related art may be difficult to wipe off.

### SUMMARY

The present disclosure has been made in consideration of the above circumstances, and an object of the present disclosure is to provide an acoustic matching member that spreads and remains on a body surface and is easily wiped off, and a method for capturing a medical image.

In order to achieve the above object, an acoustic matching member according to a first aspect of the present disclosure is an acoustic matching member that is applied to a body surface in a case where an ultrasound image is captured, in which the acoustic matching member is a mixed solution containing water and a hydrophilic polymer having a hydrophilic functional group and having 8 or more carbon atoms and a viscosity of the acoustic matching member is 5 mPa·s or more and 20000 mPa·s or less.

In an embodiment, the hydrophilic polymer is a monovalent alcohol and an aliphatic alcohol.

In an embodiment, the hydrophilic polymer is at least one of sethanol, stearyl alcohol, behenyl alcohol, or lauryl alcohol.

In an embodiment, the mixed solution is cosmetic water.

In an embodiment, the ultrasound image is captured by performing scanning on a compression member that puts a subject in a compressed state with a probe.

In addition, in order to achieve the above object, according to a second aspect of the present disclosure, a method for capturing a medical image comprises, in a state where a subject is in a state of being compressed by a compression member having a compression surface of which a region in contact with a body surface of the subject is subjected to hydrophilic processing, the subject being in a state where the body surface is coated with an acoustic matching member that is a mixed solution containing water and a hydrophilic polymer having a hydrophilic functional group and having 8 or more carbon atoms, and that has a viscosity of 5 mPa·s or more and 20000 mPa·s or less, performing ultrasound image capturing of acquiring an ultrasound image obtained by scanning a surface of the compression member opposite to the compression surface with a probe.

In an embodiment, continuously with the ultrasound image capturing, the subject in the state of being compressed by the compression member is irradiated with a radiation, and a radiographic image capturing is performed to acquire a radiographic image obtained by a radiation detector detecting the radiation transmitted through the subject.

In an embodiment, the hydrophilic processing is processing of performing hydrophilic coating on the region.

In an embodiment, the hydrophilic processing is processing of providing unevenness in the region.

In an embodiment, the processing of providing the unevenness is processing of providing a plurality of grooves having a depth of 30 µm or more and a groove width of 20 µm or more and 30 µm or less, with a surface width of 10 µm or more and 30 µm or less.

According to the present disclosure, the acoustic matching member can spread and remain on a body surface, and can be easily wiped off.

### BRIEF DESCRIPTION OF DRAWINGS

Fig. 1 is a configuration diagram schematically showing an example of an overall configuration of an image capturing system according to an embodiment.
Fig. 2 is a side view showing an example of an appearance of a mammography apparatus according to the embodiment.
Fig. 3 is a diagram showing an imaging state of an ultrasound image.
Fig. 4 is a flowchart showing an example of a flow of capturing a radiographic image and an ultrasound image using the image capturing system according to the embodiment.
Fig. 5 is a view showing a part of a compression member of a modification example.

### DETAILED DESCRIPTION

Hereinafter, an embodiment of the present invention will be described in detail with reference to the drawings. The present embodiment does not limit the present invention.

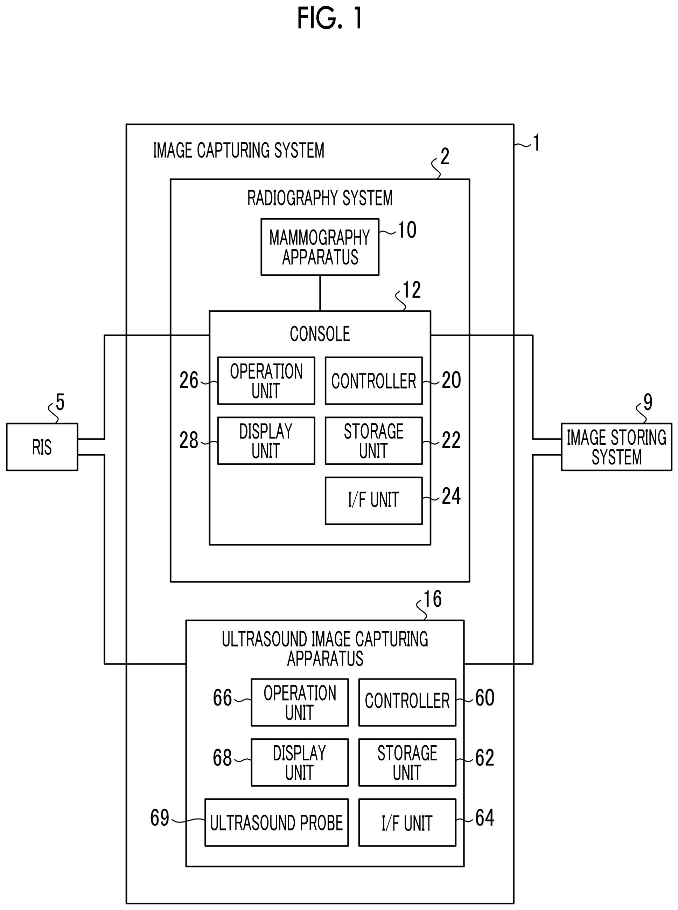

First, an example of the overall configuration of an image capturing system according to the present embodiment will be described. Fig. 1 is a configuration diagram showing an example of the overall configuration of an image capturing system 1 according to the present embodiment.

As shown in Fig. 1, an image capturing system 1 according to the present embodiment comprises a radiography system 2 and an ultrasound image capturing apparatus 16.

First, a configuration of the ultrasound image capturing apparatus 16 will be described. The ultrasound image capturing apparatus 16 is an apparatus that captures an ultrasound image with a breast of an examinee as a subject by a user, and is a so-called cart-type ultrasound image capturing apparatus. The ultrasound image capturing apparatus 16 may be an ultrasound image capturing apparatus of a so-called compact type or a handheld type other than the cart type.

The ultrasound image capturing apparatus 16 according to the present embodiment comprises a controller 60, a storage unit 62, an interface (I/F) unit 64, an ultrasound probe 69, an operation unit 66, and a display unit 68. The controller 60 comprises a central processing unit (CPU), a read only memory (ROM), a random access memory (RAM), and the like (none of which are shown), and controls the operation of the entire ultrasound image capturing apparatus 16. The ROM stores, in advance, various programs, including a program for performing control related to ultrasound image capturing, which is executed by the CPU. The RAM transitorily stores various types of data.

For example, captured ultrasound images and various other types of information are stored in the storage unit 62. As a specific example of the storage unit 62, a hard disk drive (HDD), a solid state drive (SSD), or the like is exemplified.

The operation unit 66 is used by the user to input, for example, commands or various kinds of information related to the capture of an ultrasound image. The operation unit 66 is not particularly limited. Examples of the operation unit 66 include various switches, a touch panel, a touch pen, and a mouse. The display unit 68 displays, for example, various types of information or an ultrasound image corresponding to the reception signal received from the ultrasound probe 69. Note that, the operation unit 66 and the display unit 68 may be integrated into a touch panel display.

The I/F unit 64 communicates various types of information with the RIS 5 and the image storage system 9 through wireless communication or wired communication. The ultrasound image captured by the ultrasound image capturing apparatus 16 is transmitted to the image storage system 9 through the I/F unit 64 through wireless communication or wired communication.

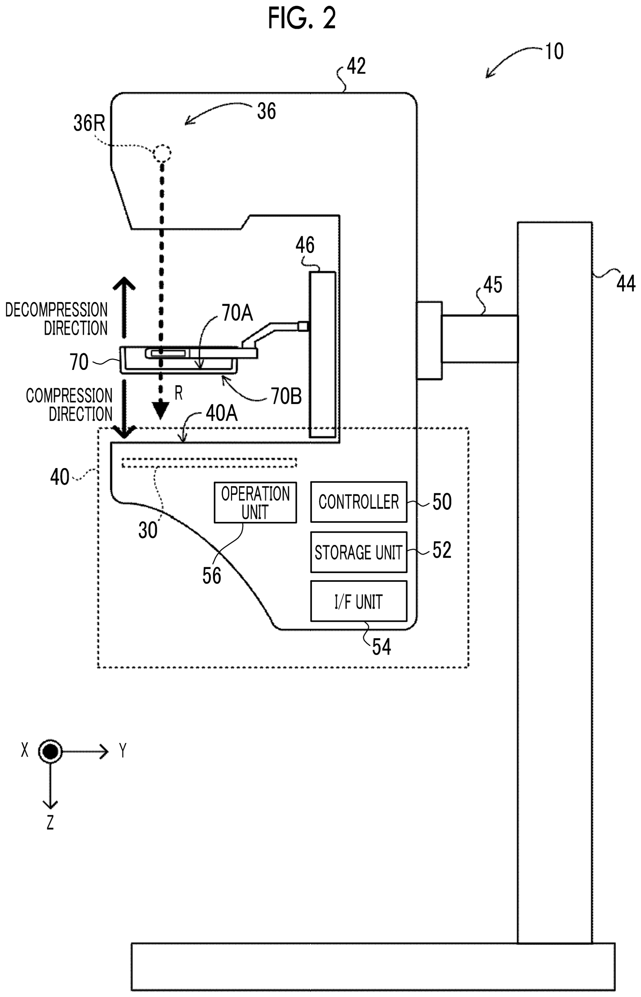

The ultrasound probe 69 is moved along an upper surface 70A (see Fig. 2, a surface opposite to the surface that comes into contact with the breast of the examinee) of the compression member 70 by the user and scans the breast with ultrasound to acquire an ultrasound image of the breast.

The ultrasound probe 69 comprises a plurality of ultrasound transducers (not shown) that are one-dimensionally or two-dimensionally arranged. Each of the ultrasound transducers transmits the ultrasound based on the applied drive signal, receives ultrasound echoes, and outputs a reception signal.

For example, each of the plurality of ultrasound transducers is a transducer configured by forming electrodes at both ends of a piezoelectric material (piezoelectric body), such as a piezoelectric ceramic typified by lead (Pb) zirconate titanate (PZT) or a polymeric piezoelectric element typified by polyvinylidene difluoride (PVDF). In a case where a pulsed or continuous wave drive signal is transmitted to apply a voltage to the electrodes of the transducer, the piezoelectric body is expanded and contracted. Pulsed or continuous wave ultrasound is generated from each transducer by the expansion and contraction and the ultrasound is synthesized to form an ultrasound beam. Further, each transducer receives the propagated ultrasound and is then expanded and contracted to generate an electric signal. The electric signal is output as an ultrasound reception signal and is input to the main body (not shown) of the ultrasound image capturing apparatus 16 through a cable (not shown).

Next, a configuration of the radiography system 2 will be described. The radiography system 2 includes a mammography apparatus 10 and a console 12.

The console 12 according to the present embodiment has a function of controlling the mammography apparatus 10 using, for example, an imaging order and various kinds of information acquired from a radiology information system (RIS) 5 through a wireless communication local area network (LAN) and commands input by the user through an operation unit 66 (see Fig. 2) of the mammography apparatus 10. Examples of the console 12 include a server computer.

The console 12 according to the present embodiment comprises a controller 20, a storage unit 22, an I/F unit 24, an operation unit 26, and a display unit 28. The controller 20 comprises a CPU, a ROM, a RAM, and the like (not shown), and controls the operation of the mammography apparatus 10. The ROM stores, in advance, various programs including a program for controlling the mammography apparatus 10 executed by the CPU. The RAM transitorily stores various types of data.

The storage unit 22 stores the captured radiographic image, various other information, and the like. Specific examples of the storage unit 22 include an HDD, an SSD, and the like.

The operation unit 26 is used for a user to input commands or various kinds of information related to the capturing of the radiographic image. The operation unit 26 is not particularly limited, and examples of the operation unit 26 include various switches, a touch panel, a touch pen, a mouse, and the like. The display unit 28 displays various kinds of information, a radiographic image captured by the mammography apparatus 10, and the like. The operation unit 26 and the display unit 28 may be integrated into a touch panel display.

The I/F unit 24 performs communication of various kinds of information to and from the image storage system 9, such as the RIS 5 and a picture archiving and communication systems (PACS), by wireless communication or wired communication. The radiographic image captured by the mammography apparatus 10 is transmitted to the image storage system 9 by wireless communication or wired communication through the I/F unit 24.

The mammography apparatus 10 according to the present embodiment irradiates the breast of an examinee as a subject with radiation R (for example, X-rays) to capture a radiographic image of the breast. Note that the mammography apparatus 10 may be an apparatus that images the breast of the examinee in a state where the examinee is sitting on a chair (including a wheelchair) or the like (sitting state) in addition to a state where the examinee is standing (standing state).

Fig. 2 is a side view showing an example of the appearance of the mammography apparatus 10 of the present embodiment. In addition, Fig. 2 is a side view showing the mammography apparatus 10 as viewed from the right side of an examinee. As shown in Fig. 2, the mammography apparatus 10 comprises a radiation source 36R, a radiation detector 30, an imaging table 40 disposed between the radiation source 36R and the radiation detector 30, and a compression member 70 that compresses the breast between the compression member 70 and the imaging table 40.

The imaging table 40 comprises a controller 50, a storage unit 52, an I/F unit 54, an operation unit 56, and the radiation detector 30. The controller 50 controls an overall operation of the mammography apparatus 10 in accordance with the control of the console 12. The controller 50 comprises a CPU, a ROM, a RAM, and the like (not shown). The ROM stores, in advance, various programs, including a program for performing control related to radiographic image capturing, which is executed by the CPU. The RAM transitorily stores various types of data.

Image data of a radiographic image and various other types of information are stored in the storage unit 52. The storage unit 52 is realized by, for example, a storage medium such as an HDD, an SSD, and a flash memory. Note that, hereinafter, "image data of a radiographic image" is simply referred to as a "radiographic image". Similarly, "image data of an ultrasound image" is simply referred to as an "ultrasound image".

The I/F unit 54 communicates various types of information with the console 12 by wired communication or wireless communication. Specifically, the I/F unit 54 receives information regarding the control of the mammography apparatus 10 from the console 12. In addition, the I/F unit 54 transmits a radiographic image to the console 12.

The operation unit 56 is a part provided on the imaging table 40 or the like and operable by a user with a hand, a foot, or the like, and is, for example, a switch, a button, a touch panel, or the like. For example, the operation unit 56 may receive a voice input from the user.

The radiation detector 30 is disposed inside the imaging table 40 and detects the radiation R transmitted through the breast which is the subject. In the mammography apparatus 10 of the present embodiment, in a case where imaging is performed, the breast of the examinee is positioned on an imaging surface 40A of the imaging table 40 by a user such as a doctor or a radiology technician. For example, the imaging surface 40A and the like with which the breast of the examinee comes into contact are made of carbon or the like in terms of the transmittance and intensity of the radiation R.

The radiation detector 30 detects the radiation R transmitted through the breast of the examinee and the imaging table 40, generates a radiographic image based on the detected radiation R, and outputs the generated radiographic image. The type of the radiation detector 30 of the present embodiment is not particularly limited. For example, the radiation detector 30 may be an indirect conversion type radiation detector that converts the radiation R into light and converts the converted light into electric charges, or may be a direct conversion type radiation detector that directly converts the radiation R into electric charges.

The radiation source 36R is provided in a radiation emitting unit 36. As shown in Fig. 2, the radiation emitting unit 36 is provided on the arm portion 42 together with the imaging table 40 and a compression unit 46. In addition, as shown in Fig. 2, the mammography apparatus 10 of the present embodiment comprises the arm portion 42, a base 44, and a shaft portion 45. The arm portion 42 is held by the base 44 so as to be movable in an up-down direction (Z-axis direction). The shaft portion 45 connects the arm portion 42 to the base 44. Further, the arm portion 42 can be relatively rotated with respect to the base 44, using the shaft portion 45 as a rotation axis.

Further, as shown in Fig. 2, the compression member 70 is attached to the compression unit 46. The compression unit 46 and the arm portion 42 can be relatively rotated with respect to the base 44 separately, using the shaft portion 45 as a rotation axis. In the present embodiment, gears (not shown) are provided in each of the shaft portion 45, the arm portion 42, and the compression unit 46. Each gear is switched between an engaged state and a disengaged state to connect each of the arm portion 42 and the compression unit 46 to the shaft portion 45. One or both of the arm portion 42 and the compression unit 46 connected to the shaft portion 45 are rotated integrally with the shaft portion 45.

The compression member 70 of the present embodiment is moved in the up-down direction (Z-axis direction) by a compression plate driving unit (not shown) provided in the compression unit 46, and compresses the breast of the examinee between the imaging table 40. As shown in Fig. 2, regarding the movement direction of the compression member 70, the direction in which the breast is compressed, in other words, the direction in which the compression member 70 approaches the imaging surface 40A is referred to as a "compression direction" and the direction in which the compression of the breast is released, in other words, the direction in which the compression member 70 approaches the radiation emitting unit 36 is referred to as a "decompression direction".

Note that the compression member 70 is not limited to a member that compresses the entire breast, but may be a member that compresses a part of the breast. In other words, the compression member 70 may be smaller than the breast. As such a compression member 70, for example, a compression member 70 used for so-called spot imaging, in which a radiographic image is captured of only a region where a lesion is present, is known.

It is preferable that the compression member 70 is optically transparent in order to check positioning or a compressed state in the compression of the breast. In addition, the compression member 70 is made of a material having high transmittance for the radiation R. Further, it is desirable that the compression member 70 is made of a material that facilitates the propagation of ultrasonic waves transmitted from the ultrasound probe 69 of the ultrasound image capturing apparatus 16. Examples of the material forming the compression member 70 include resins such as polymethylpentene, polycarbonate, acrylic, or polyethylene terephthalate. In particular, polymethylpentene is suitable as the material forming the compression member 70 since it has low rigidity, high elasticity, and high flexibility and has suitable values for acoustic impedance that affects the reflectivity of ultrasound waves and an attenuation coefficient that affects the attenuation of ultrasound waves. The member constituting the compression member 70 is not limited to the present embodiment.

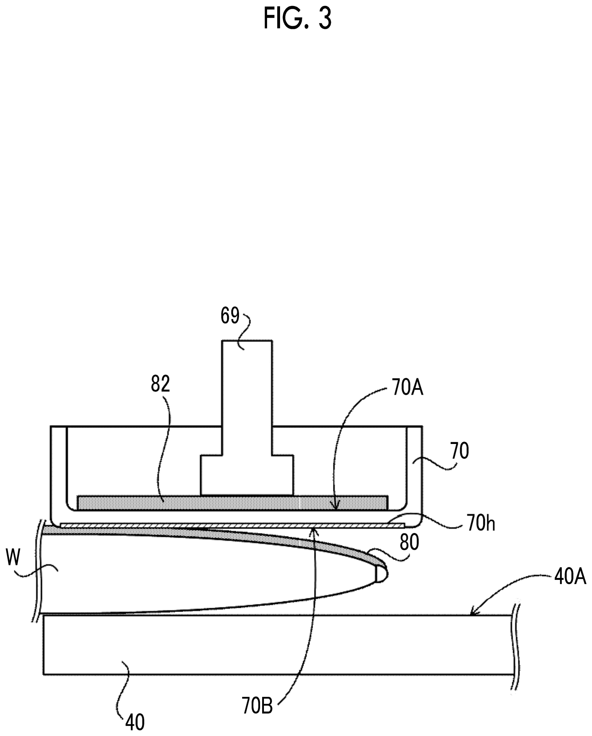

In addition, as shown in Fig. 3, a hydrophilic region 70h, which is subjected to hydrophilic processing on a region in contact with the breast W, is provided in a compression surface 70B of the compression member 70, which is a surface that comes into contact with the breast W of the examinee. The hydrophilic region 70h on the compression surface 70B may include at least a region in contact with the breast W, or may be the entire compression surface 70B.

As shown in Fig. 3, in a case where the ultrasound image is captured, the breast W placed on the imaging surface 40A of the imaging table 40 is compressed by the compression member 70 in a state where the acoustic matching member 80 is applied to the body surface that comes into contact with the compression member 70. The acoustic matching member 80 is used to suppress a difference in acoustic impedance at a boundary surface between the breast W and the compression member 70.

The acoustic matching member 80 needs to spread and remain on the body surface of the breast and the compression surface 70B. In a case where the viscosity of the acoustic matching member 80 is low, the contact angle with the body surface is large, and the acoustic matching member 80 does not spread over the entire body surface and remains as a plurality of water droplets. For example, in a case where the viscosity of pure water is about 1 mPa·s, which is measured under the measurement conditions of a measurement temperature of 20°C and a rotation speed of 1300 rotations/minute (rpm), the acoustic matching member do not spread over the entire surface of the body and remain as a plurality of water droplets. In addition, in a case where the viscosity is low, the liquid is likely to flow, and thus the acoustic matching member is unlikely to remain on the surface of the body.

On the other hand, in a case of an acoustic matching member such as echo jelly that is generally used in capturing of an ultrasound image, since the viscosity is 25000 mPa·s to 45000 mPa·s, the acoustic matching member can sufficiently spread and remain on the entire body surface. However, in a case where the viscosity is high in this way, the acoustic matching member is difficult to flow, and thus it is difficult to wipe off the acoustic matching member, and the acoustic matching member is difficult to be cleaned.

Therefore, in the acoustic matching member 80 of the present embodiment, the viscosity measured under the measurement conditions of a measurement temperature of 20°C and a rotation speed of 1300 rotations/minute (rpm) by a rotational viscometer such as a B-type viscometer is 5 mPa·s or more and 20000 mPa·s or less. By setting the viscosity to be in the above-described range, the acoustic matching member 80 can spread and remain on the entire body surface of the breast W in the compressed state by the compression member 70 and can be easily wiped off.

The acoustic matching member 80 of the present embodiment that can suppress a difference in acoustic impedance at a boundary surface between the breast W and the compression member 70 and that has the above-described viscosity is a mixed solution containing water and a hydrophilic polymer having a hydrophilic functional group and having 8 or more carbon atoms. Specifically, as the hydrophilic polymer, a monovalent alcohol and an aliphatic alcohol are preferable. In particular, as the hydrophilic polymer, at least one of sethanol (CH₃(CH₂)₁₅OH), stearyl alcohol (CH₃(CH₂)₁₇OH), behenyl alcohol (CH₃(CH₂)₂₁OH), or lauryl alcohol (CH₃(CH₂)₁₁OH) is preferable.

As the mixed solution used as the acoustic matching member 80, "cosmetic water" can be used. It is preferable that the cosmetic water does not contain anhydrous ethanol referred to as "alcohol-free" or "non-alcohol".

It is necessary that the acoustic matching member 80 is sufficiently spread on the compression surface 70B of the compression member 70 and air bubbles or the like are not mixed. Therefore, in the compression surface 70B of the compression member 70, it is preferable that at least a region in contact with the breast W is hydrophilic. In other words, it is preferable that the contact angle of the acoustic matching member 80 with respect to at least the region in contact with the breast W of the compression surface 70B is small. However, the above-described resins and the like that can be used as a material for the compression member 70 are often hydrophobic. Therefore, as shown in Fig. 3, the compression surface 70B of the compression member 70 according to the present embodiment has a hydrophilic region 70h that is subjected to hydrophilic processing in a region in contact with the breast W.

Specifically, the hydrophilic region 70h of the compression surface 70B of the compression member 70 according to the present embodiment is coated with a hydrophilic coating. The type of the hydrophilic coating and the method of performing the hydrophilic coating are not limited.

Examples of the method of performing the hydrophilic coating include a method of performing a plasma treatment of forming a functional group (OH group) having a high hydrophilicity on a surface of the hydrophilic region 70h using plasma and activating and planarizing the surface to chemically improve the hydrophilicity.

In addition, examples of the method of performing the hydrophilic coating include a method of improving the hydrophilicity by coating a surfactant on the hydrophilic region 70h to reduce the interfacial tension at the interface between the acoustic matching member 80 and the compression surface 70B.

Further, examples of the method of performing the hydrophilic coating include a method of improving the hydrophilicity by coating a hydrophilic binder on the hydrophilic region 70h to improve the adhesiveness between the acoustic matching member 80 and the compression surface 70B.

In the image capturing system 1 of the present embodiment, the capturing of the radiographic image and the capturing of the ultrasound image are continuously performed in a state where the breast W is put in a compressed state by the compression member 70. In the present embodiment, since the capturing of the radiographic image and the capturing of the ultrasound image are continuously performed without completely releasing the compression by the compression member 70, the capturing of the radiographic image is performed in a state where the acoustic matching member 80 is provided. Therefore, the above described the hydrophilic processing performed on the hydrophilic region 70h of the compression surface 70B of the compression member 70, and the material of the acoustic matching member 80 are specifically selected in consideration of the influence on the radiographic image. For example, it is preferable that the hydrophilic polymer is composed of atoms having an atomic number of 11 or less as the acoustic matching member 80.

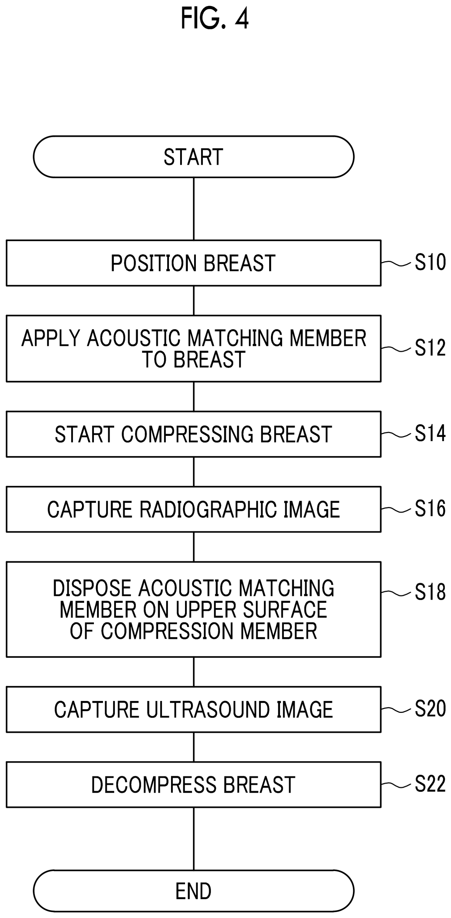

Next, a flow of capturing a radiographic image and an ultrasound image using the image capturing system 1 will be described. Fig. 4 is a flowchart showing an example of a flow of capturing the radiographic image and the ultrasound image using the image capturing system 1 according to the present embodiment.

First, in Step S10 of Fig. 9, the user positions the breast W of the examinee, which is the subject, on the imaging surface 40A of the imaging table 40.

In the next Step S12, as shown in Fig. 3, the user applies the acoustic matching member 80 to the body surface of the breast W. In a case where the application of the acoustic matching member 80 is completed, the user gives a command to compress the breast W by the operation unit 66.

In the next Step S14, the controller 60 of the mammography apparatus 10 starts compressing the breast W by the compression member 70. Specifically, in a case where a command to compress the breast W is received, the controller 60 moves the compression member 70 in the compression direction, and puts the breast W into a compressed state between the compression member 70 and the imaging surface 40A of the imaging table 40. Accordingly, the breast W is in contact with the hydrophilic region 70h of the compression surface 70B of the compression member 70 via the acoustic matching member 80.

In the next Step S16, the mammography apparatus 10 captures the radiographic image of the breast W. Specifically, the user operates an irradiation switch included in the operation unit 66 to irradiate the breast W with the radiation R emitted from the radiation source 36R and to capture the radiographic image with the radiation detector 30. The radiographic image captured by the mammography apparatus 10 is output to the console 12, is output from the console 12 to the image storage system 9 at a predetermined timing, and is stored in the image storage system 9.

In a case where the capturing of the radiographic image ends, in next Step S18, as shown in Fig. 3, the user disposes the acoustic matching member 82 on the upper surface 70A of the compression member 70. The acoustic matching member 82 is used to suppress a difference in acoustic impedance at a boundary surface between the compression member 70 and the ultrasound probe 69. For example, in the present embodiment, a general-purpose echo jelly, a gel pad, or the like is used. It should be noted that, unlike the aspect shown in Fig. 3, in a case where the acoustic matching member 82 is provided on the surface of the ultrasound probe 69 that comes into contact with the compression member 70, this step is omitted.

In next Step S20, the technician scans the breast W compressed by the compression member 70 with the ultrasound probe 69 of the ultrasound image capturing apparatus 16 along the upper surface 70A of the compression member 70 on which the acoustic matching member 82 is provided to capture a plurality of ultrasound images. The ultrasound image captured by the ultrasound image capturing apparatus 16 is temporarily stored in the storage unit 62, is then output from the ultrasound image capturing apparatus 16 to the image storage system 9 at a predetermined timing, and is stored in the image storage system 9.

In a case where the capturing of the ultrasound image ends, in the next Step S22, the compression of the breast W using the compression member 70 is released. Specifically, the user gives a command to release compression using the operation unit 66. In a case where the command to release the compression of the breast W is received, the controller 50 moves the compression member 70 in the decompression direction, moves the compression member 70 in a direction away from the imaging surface 40A of the imaging table 40, and releases the compression of the breast W by the compression member 70.

In this way, in a case where the processing of Step S22 is completed, the capturing of the radiographic image and the ultrasound image is completed.

In a case where the compression of the breast W is released, the acoustic matching member 80 applied to the body surface of the breast W is wiped off by the examinee. In addition, the acoustic matching member 80 adhering to the compression surface 70B of the compression member 70 and the acoustic matching member 82 adhering to the upper surface 70A are cleaned.

### Modification Example

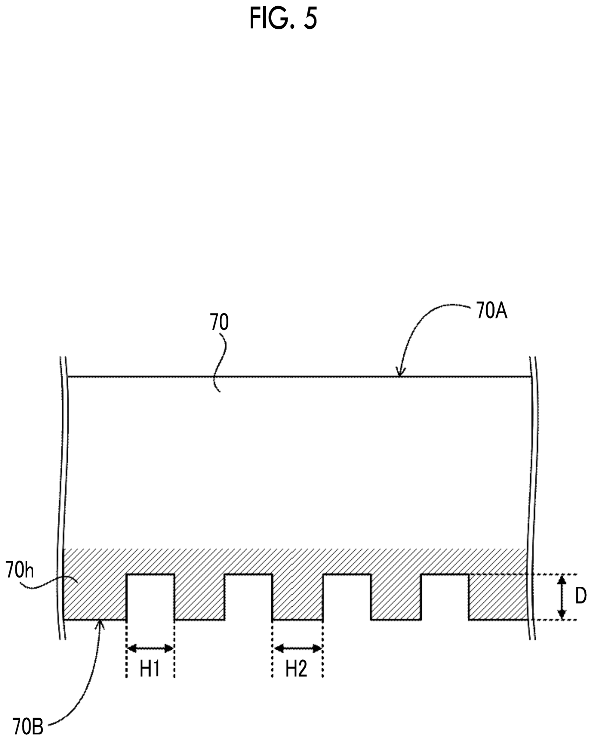

A modification example of the hydrophilic processing performed on the hydrophilic region 70h of the compression surface 70B of the compression member 70 will be described with reference to Fig. 5. Fig. 5 shows a part of the compression member 70.

It is known by, for example, the Wenzel model that the wettability, that is, the hydrophilicity is determined based on the surface area of the interface between the solid and the liquid, and the hydrophilicity may be increased by forming an uneven structure on the hydrophilic interface to increase the surface area.

Therefore, as shown in Fig. 5, in the hydrophilic region 70h of the compression surface 70B of the compression member 70 according to the present modification example, a plurality of grooves are provided as the hydrophilic processing to provide unevenness in the hydrophilic region 70h.

As described above, the smaller the contact angle between the acoustic matching member 80 and the hydrophilic region 70h of the compression surface 70B, the higher the hydrophilicity. In the uneven structure, as the groove depth D increases, the contact angle decreases. In addition, as the groove width H1 and the surface width H2 increase, the surface approaches flatness. Therefore, in consideration of the affinity with the acoustic matching member 80, in a case where the grooves are provided as the uneven structure as in the present modification example, it is preferable that the plurality of grooves in which the groove depth D is 30 µm or more and the groove width H1 is 20 µm or more and 30 µm or less are provided at the surface width H2 of 10 µm or more and 30 µm or less.

Examples of processing of providing such an uneven structure include so-called emboss processing. In addition, a specific processing method is not limited. For example, a method of directly forming the hydrophilic region 70h of the compression surface 70B of the compression member 70 in an uneven shape may be used. In addition, for example, a method of applying a hydrophilic coating agent or the like to the hydrophilic region 70h to impart an uneven shape to the surface may be used.

As in the present modification example, the transparency of the compression member 70 may be reduced by performing the unevenness processing on the hydrophilic region 70h of the compression surface 70B of the compression member 70. However, in a case of compressing the breast W, since the breast W is compressed in a state where the acoustic matching member 80 is applied to the body surface, the acoustic matching member 80 is in a state of being filled in the groove portion having the uneven structure, and the transparency can be improved. As described above, in a case where the breast W is compressed, the transparency can be improved, and thus the user can sufficiently visually recognize the state of the breast W through the compression member 70.

As described above, the acoustic matching member 80 of the above-described embodiment is an acoustic matching member that is applied to a body surface in a case where an ultrasound image is captured, and is a mixed solution containing water and a hydrophilic polymer having a hydrophilic functional group and having 8 or more carbon atoms, and that has the viscosity 5 mPa·s or more and 20000 mPa·s or less.

With such a configuration, the viscosity can be set to an appropriate viscosity, and thus, the acoustic matching member 80 can spread and remain on the body surface, and can be easily wiped off.

In addition, in the above-described embodiment, since the cosmetic water is used as the acoustic matching member, it is possible to protect the body surface, for example, to give moisture to the breast which is the subject.

In the present embodiment, as in the flow shown in Fig. 4, continuously capturing the radiographic image and the ultrasound image while the breast W is in a compressed state by the compression member 70 may be referred to as "continuous imaging". The order of capturing the radiographic image (Step S16 in Fig. 4) and capturing the ultrasound image (Step S20 in Fig. 4) is not limited. However, from the viewpoint of reducing the compression time of the breast W, it is preferable to perform the capturing of the radiographic image first as in the present embodiment. In addition, the compression force with which the breast W is compressed by the compression member 70 may be weakened as long as it can be considered that there is no change in the compressed states of the breast W between the capturing of the radiographic image and the capturing of the ultrasound image U. For example, the compression force by the compression member 70 may be weakened as long as it can be considered that no change has occurred in the degree of expansion of the mammary glands of the breast W.

In addition, the configurations and operations of the image capturing system 1, the radiography system 2, the mammography apparatus 10, the console 12, the ultrasound image capturing apparatus 16, and the like described in each of the above-described embodiments are merely examples and it goes without saying that they can be changed according to the situation without departing from the gist of the present invention. In addition, it is needless to say that the embodiment and the modification example may be appropriately combined.

Regarding the above embodiments, the following Supplementary Notes are further disclosed.

### Supplementary Note 1

An acoustic matching member that is applied to a body surface in a case where an ultrasound image is captured,
wherein the acoustic matching member is a mixed solution containing:
   water; and
   a hydrophilic polymer having a hydrophilic functional group and having 8 or more carbon atoms, and
a viscosity of the acoustic matching member is 5 mPa·s or more and 20000 mPa·s or less.

### Supplementary Note 2

The acoustic matching member according to Supplementary Note 1,
wherein the hydrophilic polymer is a monovalent alcohol and an aliphatic alcohol.

### Supplementary Note 3

The acoustic matching member according to Supplementary Note 1 or 2,
wherein the hydrophilic polymer is at least one of sethanol, stearyl alcohol, behenyl alcohol, or lauryl alcohol.

### Supplementary Note 4

The acoustic matching member according to any one of Supplementary Notes 1 to 3, wherein the mixed solution is cosmetic water.

### Supplementary Note 5

The acoustic matching member according to any one of Supplementary Notes 1 to 4,
wherein the ultrasound image is captured by performing scanning on a compression member that puts a subject in a compressed state with a probe.

### Supplementary Note 6

The acoustic matching member according to any one of Supplementary Notes 1 to 5,
wherein the hydrophilic polymer is composed of atoms having an atomic number of 11 or less.

### Supplementary Note 7

A compression member that compresses a subject in a case where an ultrasound image is captured, the compression member comprising:
a compression surface of which a region in contact with the subject is subjected to hydrophilic processing.

### Supplementary Note 8

The compression member according to Supplementary Note 7,
wherein the hydrophilic processing is processing of performing hydrophilic coating on the region.

### Supplementary Note 9

The compression member according to Supplementary Note 7 or 8,
wherein the hydrophilic processing is processing of providing unevenness in the region.

### Supplementary Note 10

The compression member according to Supplementary Note 9,
wherein the processing of providing the unevenness is processing of providing a plurality of grooves having a depth of 30 µm or more and a groove width of 20 µm or more and 30 µm or less, with a surface width of 10 µm or more and 30 µm or less.

### Supplementary Note 11

A method for capturing a medical image, comprising:
in a state where a subject is in a state of being compressed by a compression member having a compression surface of which a region in contact with a body surface of the subject is subjected to hydrophilic processing, the subject being in a state where the body surface is coated with an acoustic matching member that is a mixed solution containing water and a hydrophilic polymer having a hydrophilic functional group and having 8 or more carbon atoms, and that has a viscosity of 5 mPa·s or more and 20000 mPa·s or less,
performing ultrasound image capturing of acquiring an ultrasound image obtained by scanning a surface of the compression member opposite to the compression surface with a probe.

### Supplementary Note 12

The method for capturing a medical image according to Supplementary Note 11,
wherein, continuously with the ultrasound image capturing,
the subject in the state of being compressed by the compression member is irradiated with a radiation, and
a radiographic image capturing is performed to acquire a radiographic image obtained by a radiation detector detecting the radiation transmitted through the subject.

### Supplementary Note 13

The method for capturing a medical image according to Supplementary Note 11 or 12,
wherein the hydrophilic processing is processing of performing hydrophilic coating on the region.

### Supplementary Note 14

The method for capturing a medical image according to any one of Supplementary Notes 11 to 13,
wherein the hydrophilic processing is processing of providing unevenness in the region.

### Supplementary Note 15

The method for capturing a medical image according to any one of Supplementary Note 14,
wherein the processing of providing the unevenness is processing of providing a plurality of grooves having a depth of 30 µm or more and a groove width of 20 µm or more and 30 µm or less, with a surface width of 10 µm or more and 30 µm or less.

### Explanation of References

1: image capturing system
2: radiography system
5: RIS
9: image storage system
10: mammography apparatus
12: console
16: ultrasound image capturing apparatus
20, 50, 60: controller
22, 52, 62: storage unit
24, 54, 64: I/F unit
26, 56, 66: operation unit
28, 68: display unit
30: radiation detector, 30A: detection surface
36: radiation emitting unit, 36R: radiation source
40: imaging table, 40A: imaging surface
42: arm portion
44: base
45: shaft portion
46: compression unit
69: ultrasound probe
70: compression plate, 70A: upper surface, 70B: compression surface, 70h: hydrophilic region
80, 82: acoustic matching member
D: groove depth
H1: groove width, H2: surface width
R: radiation
W: breast

## Claims

1. A method for capturing a medical image, comprising:
in a state where a subject is in a state of being compressed by a compression member (70) having a compression surface (70B) of which a region in contact with a body surface of the subject is subjected to hydrophilic processing, the subject being in a state where the body surface is coated with an acoustic matching member (80, 82) that is a mixed solution containing water and a hydrophilic polymer having a hydrophilic functional group and having 8 or more carbon atoms, and that has a viscosity of 5 mPa·s or more and 20000 mPa·s or less,
performing ultrasound image capturing of acquiring an ultrasound image obtained by scanning a surface (70A) of the compression member (70) opposite to the compression surface (70B) with a probe (69).

2. The method for capturing a medical image according to claim 1, wherein continuously with the ultrasound image capturing,
the subject in the state of being compressed by the compression member (70) is irradiated with radiation, and
radiographic image capturing is performed to acquire a radiographic image obtained by a radiation detector (30) detecting the radiation transmitted through the subject.

3. The method for capturing a medical image according to claim 1, wherein the hydrophilic processing is processing of performing hydrophilic coating on the region.

4. The method for capturing a medical image according to claim 1, wherein the hydrophilic processing is processing of providing unevenness in the region.

5. The method for capturing a medical image according to claim 4, wherein the processing of providing the unevenness is processing of providing a plurality of grooves having a depth of 30 µm or more and a groove width of 20 µm or more and 30 µm or less, with a surface width of 10 µm or more and 30 µm or less.
